# EUROPEAN PATENT SPECIFICATION

(11) **EP 3 511 001 B1**
(45) Date of publication and mention of the grant of the patent: **22.09.2021**
(21) Application number: 18202096.6
(22) Date of filing: 23.10.2018
(51) Int. Cl.: A61K 31/4418, A61K 9/16, A61K 9/20, A61K 9/48

(54) **PIRFENIDONE-CONTAINING TABLET AND CAPSULE FORMULATION**
PIRFENIDONHALTIGE TABLETTE UND KAPSELFORMULIERUNG
COMPRIMÉ CONTENANT DE LA PIRFÉNIDONE ET FORMULATION DE CAPSULE

(30) Priority: 12.01.2018 IN 201811001461
(43) Date of publication of application: 17.07.2019
(73) Proprietor: Alfred E. Tiefenbacher (GmbH & Co. KG), 22767 Hamburg (DE); Laurus Labs Limited, Hyderabad 500034 (IN)
(72) Inventor: Gujjar, Chaitanya Yogananda, 502 319 Telangana State (IN); Uppala, Susheel Prakash, 502 319 Telangana State (IN); Dhanala, Harish, 502 319 Telangana State (IN); Donga, Nani Prasad, 502 319 Telangana State (IN); Bandla, Srimannarayana, 502 319 Telangana State (IN); Rallabandi, Bala Ramesha Chary, 502 319 Telangana State (IN); Velaga, Siva Rama Krishna, 500078 Hyderabad (IN); Schlehahn, Hendrik, 22767 Hamburg (DE)
(74) Representative: Hamm&Wittkopp Patentanwälte PartmbB

(56) References cited:
- EP-A1- 2 756 840
- WO-A1-2017/172602
- WO-A2-2007/038315
- CN-A- 102 846 569

## Description

### FIELD OF INVENTION

The present invention relates to granules containing pirfenidone and a sugar alcohol, and, furthermore, to the use of the granules for the preparation of an immediate-release tablet or capsule.

### BACKGROUND OF INVENTION

Pirfenidone is marketed under the tradename Esbriet^{®} in the form of film-coated tablets, which contain 267, 534 or 801 mg pirfenidone, or as a hard capsule containing 267 mg pirfenidone, for the treatment of idiopathic pulmonary fibrosis (IPF). The recommended daily maintenance dosage of Esbriet^{®} is 801 mg three times daily for a total of 2403 mg/day. Upon initiation of treatment, titration to the full dosage of 2403 mg/day over a 14-day period is required (days 1-7, 267 mg three times daily; days 8-14, 534 mg three times daily; days 15 onward, 801 mg three times daily).

The Esbriet^{®} tablets and capsule provide for an immediate-release of the drug. The capsule contains, besides the drug, microcrystalline cellulose, croscarmellose sodium, povidone and magnesium stearate, while the tablet contains, in the core, the drug, microcrystalline cellulose, colloidal anhydrous silica, povidone K30, croscarmellose sodium and magnesium stearate. Pirfenidone is a crystalline powder and sparingly soluble in 1.0NHCl, water and 1.0 N NaOH; the dissolution in water is pH independent (19 mg/ml at 25°C). Pirfenidone has poor flowability characteristics, and in view of the fact that the Esbriet^{®} capsule and tablets contain high concentrations of pirfenidone, in order to provide a tablet and capsule size that is suitable for oral administration, the state of the art focuses on improving the processability of the drug. The Esbriet^{®} capsule and tablets are prepared by using wet-granulation.

CN 102846569 discloses the preparation of a tablet composition comprising pirfenidone, lactose, microcrystalline cellulose, crosslinked sodium carboxymethyl cellulose, hydroxypropyl cellulose and magnesium stearate. EP-A-1 356 816 describes the preparation of a tablet containing 200 mg pirfenidone, in which the drug, lactose and a part of the contained carmellose calcium is subjected to wet-granulation using an aqueous solution of hydroxypropyl cellulose as a granulating fluid, followed by mixing the obtained granules with the remaining part of carmellose calcium and magnesium stearate, and subjecting the mixture to compression. Subsequently, the tablet core is coated with a film. This tablet is marketed in Japan under the tradename Pirespa^{®} for the treatment of IPF.

It is stated in WO 2007/038315 that the Pirespa^{®} tablet core contains about 70% pirfenidone and that a higher drug content would be desirable. WO 2007/038315 relates to a capsule formulation containing 70-95 % pirfenidone and 5-30 % pharmaceutical excipients. This application relates to the Esbriet^{®} capsule, which may be prepared by milling a mixture of croscarmellose sodium, microcrystalline cellulose and pirfenidone, and subjecting the obtained mixture to wet-granulation using an aqueous povidone solution as granulating liquid. Subsequently, the granules are mixed with an additional amount of croscarmellose sodium and magnesium stearate, and, thereafter, the obtained mixture is filled into a capsule.

EP-A-2 735 306 discloses an extended-release tablet containing pirfenidone. The tablet is prepared by direct compression, in which pirfenidone and silicon dioxide are mixed in order to improve the flowability of the drug, followed by adding microcrystalline cellulose, low-viscosity hydroxypropyl methyicellulose (HPMC), high-viscosity HPMC and sodium stearyl fumarate, and subjecting the obtained mixture to compression. The low and high-viscosity HPMCs form the extended-release matrix, whereby the microcrystalline cellulose improves the tablet hardness.

WO 2017/172602 relates to the Esbriet^{®} tablet. It is stated in the application that the particle size of pirfenidone can vary between the suppliers and that is was found that these particle size variations affect the hardness of the tablet. However, the particle size distribution of pirfenidone has no influence on the drug release characteristics, if the Dv90 value is adjusted to 50-150 µm. Rather, the drug release characteristics depend on the solid fraction of the tablet (the ratio of the tablet's apparent density and true density): the higher the solid fraction, the longer the disintegration time.

The tablet is prepared by subjecting pirfenidone, a filler (preferably microcrystalline cellulose) and a glidant (preferably silica) to wet-granulation using an aqueous binder (preferably povidone) solution. The obtained granules are subsequently mixed with a disintegrant (preferably croscarmellose sodium), a lubricant (preferably magnesium stearate) and a glidant (preferably silica). Finally, the obtained mixture is subjected to compression, and the obtained tablet core is film-coatcd. The intragranular glidant is required to improve the flowability of pirfenidone.

### OBJECT OF THE INVENTION

It was an objective of the present invention to provide an immediate-release tablet or capsule for oral administration containing pirfenidone, which may contain the drug in a high concentration of at least 70 % by weight and which can be prepared from a drug formulation that exhibits good flow properties. This objective is attained by the subject matter as defined in the claims.

### SUMMARY OF THE INVENTION

The present invention relates to granules containing pirfenidone and a sugar alcohol, and, furthermore, to the use of the granules for the preparation of an immediate-release tablet or capsule,

### DETAILED DESCRIPTION

The unit dosage form of the present invention is an immediate-release tablet or an immediate-release capsule for oral administration; preferably the tablet is a film-coated tablet. These unit dosage forms contain granules containing pirfenidone, a sugar alcohol, optionally a disintegrant and optionally a further pharmaceutical excipient, wherein the weight ratio of the sugar alcohol to pirfenidone is 2 : 100 to 30 : 100. Furthermore these unit dosage forms may contain pirfenidone in a total amount of 267 mg, 534 mg or 801 mg. It was found that a glidant is not required in order to improve the flowability of pirfenidone, if the powder mixture to be subjected to granulation contains a sugar alcohol. Thus, in a preferred embodiment of the present invention, the granules do not contain a glidant.

It is preferred that the weight ratio of the sugar alcohol to pirfenidone is 3 : 100 to 25 : 100, wherein the granules to be filled into the capsule contain the sugar alcohol and pirfenidone preferably in a weight ratio of 5 : 100 to 15 : 100, more preferred 8 : 100 to 12 : 100 and most preferred is 10.5:100 (sugar alcohol: pirfenidone), and wherein the granules for tablets contain the sugar alcohol and pirfenidone more preferred in a weight ratio of 3.1:100 (sugar alcohol : pirfenidone).

The further pharmaceutical excipient, optionally contained in the granules of the present invention, may be selected from a binder and a filler. It is preferred that the granules contained in the tablet of the present invention are prepared by wet-granulation, in which case a binder is preferably contained in the granules. The preferred method for preparing the granules contained in the capsule of the present invention is dry-granulation, in which case no binder is required. It was found that it is difficult to fill granules prepared by wet-granulation into size "1" capsules, but larger capsules are less convenient to the patient upon swallowing. It is preferred that the granules of the present invention consist of pirfenidone, a disintegrant, a sugar alcohol, and optionally a binder and optionally a filler.

Typically, the sugar alcohol contained in the granules of the present invention is a C₄-₁₂ sugar alcohol, such as erythritol, xylitol, sorbitol, mannitol, maltitol, lactitol and isomalt. The preferred sugar alcohol contained in the granules of the tablet is mannitol, while the preferred sugar alcohol contained in the granules of the capsule is isomalt.

It is desired that the tablet core has a minimum hardness in order to remain intact through post-compaction processes, such as coating, transport/handling and packaging. The tablet core hardness for the 267 mg strength should be at least 60 N, and, for the 543 mg and 801 mg strengths, a hardness of at least 90 N and at least 120 N, respectively, is desired. It was found that a desired minimum tablet core hardness, and thus the tablet hardness, could not be achieved if a sugar alcohol is not present in the granules.

Examples of disintegrants include croscarmellose sodium, sodium starch glycolate, polyvinylpolypyrrolidone (crospovidone), carmellose sodium, carmellose potassium, polacrilin sodium, polacrilin potassium, silicified microcrystalline cellulose and low-substituted hydroxypropyl cellulose, whereby croscarmellose sodium is preferred.

The further pharmaceutical excipient contained in the tablet and capsule of the present invention may be selected from fillers, binders, glidants and lubricants.

Examples of fillers include microcrystalline cellulose, calcium hydrogen phosphate (anhydrous or dihydrate), lactose (anhydrous or monohydrate), dextrose, calcium carbonate, starch, pregelatinized starch, magnesium carbonate, silicified microcrystalline cellulose and powder cellulose, whereby microcrystalline cellulose is preferred.

Examples of binders include hydroxymethyl cellulose, hydroxypropyl cellulose, hydroxypropyl methylcellulose (Hypromellose), guar gum, maltodextrin, corn starch, polyvinylpyrrolidone (povidone) and vinylpyrrolidone/vinyl acetate copolymer (copovidone), wherein povidone is preferred.

Examples of glidants include silicon dioxide (silica), e.g. fumed (colloidal) silicon dioxide, talc and magnesium silicate, wherein silicon dioxide is preferred.

Examples of lubricants include magnesium stearate, calcium stearate, zinc stearate, stearic acid, sodium stearyl fumarate and glyceryl dibehenate, wherein the capsule preferably contains magnesium stearate and the tablet preferably contains sodium stearyl fumarate.

The capsule of the present invention is preferably prepared by a dry-granulation process comprising the steps:
i) preparing a mixture containing pirfenidone, a sugar alcohol, optionally a first disintegrant and optionally a further first pharmaceutical excipient,
ii) subjecting the mixture obtained in step (i) to compaction,
iii) milling the compacted material obtained in step (ii) to obtain granules, wherein the weight ratio of the sugar alcohol to pirfenidone in the obtained granules is 2 : 100 to 30 : 100,
iv) optionally mixing the granules with a second disintegrant and/or a further second pharmaceutical excipient, and
v) filling the granules obtained in step (iii) or the mixture obtained in step (iv) into a capsule.

The dry-granulation process may be performed by roller compaction or slugging, wherein roller compaction is preferred. The further second pharmaceutical excipient is preferably a lubricant.

In a preferred embodiment of the process of the present invention, the capsule contains a mixture consisting of granules made from pirfenidone, a disintegrant and a sugar alcohol, and a lubricant, and the process comprises the steps:
i) preparing a mixture consisting of pirfenidone, a disintegrant and a sugar alcohol,
ii) subjecting the mixture obtained in step (i) to compaction,
iii) milling the compacted material obtained in step (ii) to obtain granules,
iv) mixing the granules obtained in step (iii) with a lubricant, and
v) filling the mixture obtained in step (iv) into a capsule.

Preferably, the disintegrant is croscarmellose sodium, the sugar alcohol is isomalt and the lubricant is magnesium stearate.

The optionally film-coated tablet of the present invention, which contains pirfenidone, a sugar alcohol, optionally a disintegrant and optionally a further pharmaceutical excipient, is preferably prepared by wet-granulation comprising the steps:
i) preparing a mixture containing pirfenidone, a sugar alcohol, optionally a first disintegrant and optionally a further first pharmaceutical excipient,
ii) subjecting the mixture obtained in step (i) to wet granulation using an aqueous granulating liquid, wherein the weight ratio of the sugar alcohol to pirfenidone in the obtained granules is 2 : 100 to 30 : 100,
iii) optionally mixing the granules obtained in step (ii) with a second disintegrant and/or a further second pharmaceutical excipient,
iv) subjecting the granules obtained in step (ii) or the mixture obtained in step (iii) to compression to obtain the tablet.

It is preferred that the first further pharmaceutical excipient used in method step (i) is a filler, while the second pharmaceutical excipient used in method step (iii) may be selected from a filler, a glidant and a lubricant. It is preferred that, for the wet-granulation step (ii), an aqueous granulating liquid containing a binder, which is preferably a binder-containing aqueous solution, is used.

According to a preferred embodiment of the present invention, the tablet or the tablet core consists of a mixture consisting of granules made from pirfenidone, a first disintegrant, a sugar alcohol, a binder and optionally a first filler, a second disintegrant, a second filler, a glidant and a lubricant, wherein the process comprises the steps:
i) preparing a mixture consisting of pirfenidone, a first disintegrant, a sugar alcohol and optionally a first filler,
ii) subjecting the mixture obtained in step (i) to wet granulation using an aqueous binder-containing solution as granulating liquid,
iii) mixing the granules obtained in step (ii) with a second disintegrant, a second filler, a glidant and a lubricant,
iv) subjecting the mixture obtained in step (iii) to compression to obtain the tablet.

The first and second disintegrant may be identical. In a preferred embodiment of the process of the present invention, the first and second disintegrant is croscarmellose sodium, the sugar alcohol is mannitol, the optionally contained first filler and the second filler are microcrystalline cellulose, the glidant is silicon dioxide and the lubricant is sodium stearyl fumarate.

The compressed tablets may optionally be coated with a film coating polymer. Examples of polymers for film coating are polyvinyl alcohol, hydroxypropyl methylcellulose, hydroxypropyl cellulose, whereby polyvinyl alcohol is preferred. The film coating layer may further comprise plasticizer and colorants. Preferably the film coating layer comprises polyvinyl alcohol, titanium dioxide, macrogol (PEG), talc and one or more colorants (e.g. iron oxide). Ready made commercially available film coating systems containing polyvinyl alcohol polymers, like Opadry^{®} Clear 06F590004, Opadry^{®} Pink OY-S-34907, Opadry^{®} II Purple 85F500082, Opadry^{®} II Purple 85F500083, Opadry^{®} Yellow 85F520311, Opadry^{®} II Brown 85F565071 and Opadry^{®} Orange 85F530178, can be used.

It was found that the particle size of pirfenidone affects the hardness of the tablet: the higher the particle size of pirfenidone, the lower the hardness of the tablet. It was found that the desired hardness could not be achieved if the utilized pirfenidone has a particle size distribution Dᵥ90 of more than 400 µm. Thus, the particle size distribution of pirfenidone is preferably adjusted to Dᵥ90 = 50-300 µm, preferably 100-200 µm and most preferred about 140-170 µm (determined by using Mastersizer 2000).

The optionally film-coated tablet and capsule of the present invention preferably contains pirfenidone in an amount of at least 70 % by weight. Preferably the capsule of the present invention comprises 80-90 % by weight of pirfenidone, 2-10 % by weight of a disintegrant, 5-15 % by weight of a sugar alcohol, 0-10 % by weight of a binder and 0.1-5 % by weight of a lubricant, for example, 84.8 % by weight of pirfenidone, 6.0 % by weight of a disintegrant (e.g. croscamellose sodium), 8.9 % by weight of a sugar alcohol (e.g. isomalt) and 0.3 % by weight of a lubricant (e.g. magnesium stearate). Preferably the tablet core of the present invention comprises 85-90 % by weight of pirfenidone, 1-10% by weight of a disintegrant, 1-10% by weight of a sugar alcohol, 0.5-7 % by weight of a binder, 1-10 % by weight of a filler, 0.1-5 % by weight of a lubricant and 0.1-5 % by weight of a glidant. More preferred the tablet core of the present invention comprises 85-90 % by weight of pirfenidone, 1-5 % by weight of a disintegrant, 1-5 % by weight of a sugar alcohol, 0.5-5 % by weight of a binder, 1-8 % by weight of a filler, 0.1-3 % by weight of a lubricant and 0.1-3 % by weight of a glidant, for example, 87 % by weight of pirfenidone, 2.2 % by weight of a disintegrant (e.g. croscarmellose sodium) and 3.2 % by weight of a sugar alcohol (e.g. mannitol) or 2.7 % by weight of disintegrant (e.g. croscarmellose sodium) and 2.7 % by weight of sugar alcohol (e.g. mannitol), 2.2 % by weight of a binder (e.g. povidone), 4.5 % by weight of a filler (e.g. microcrystalline cellulose), 0.6 % by weight of a lubricant (e.g. sodium stearyl fumarate) and 0.3 % by weight of a glidant (e.g. colloidal silicon dioxide), Preferably the capsule of the present invention comprises 267 mg of pirfenidone, whereas the tablet core of the present invention preferably comprises pirfenidone in a total amount of 267 mg, 534 mg or 801 mg.

The following examples are intended to further illustrate the present invention.

### Examples

In the examples, crystalline pirfenidone having a particle size distribution of Dᵥ90= 100-200 µm was used. Particle size distribution was determined by using laser diffraction with a Mastersizer 2000 (by Malvern Intr. Ltd.) in dry dispersion mode. Dᵥ90 corresponds to the particle size at 90% of the cumulative volume distribution. The dissolution tests were performed according to general chapter (711) DISSOLUTION of USP40-NF35 and the US FDA recommended dissolution method for pirfenidone capsule: apparatus II (paddle; with sinker in case of capsules), speed: 50 rpm, medium: deionized water, volume: 1000 ml, sampling times: 5, 10, 15, 20, 30, and 45 min as well as optionally 60 min.

Bulk density and tapped density were determined according to general chapter (616) BULK DENSITY AND TAPPED DENSITY OF POWDERS of USP40-NF35. Hardness was determined according to European Pharmacopeia 9.2, 2.9.8. Disintegration time was determined according to general chapter (701) DISINTEGRATION of USP40-NF35, Friability was determined according to general chapter (1216) TABLET FRIABILITY of USP40-NF35. Compressibility Index and Hausner's Ratio were calculated from tapped and bulk density (Compressibility Index = (tapped density - bulk density) / tapped density ^{∗}100; Hausner's Ratio = tapped density / bulk density).

### Examples 1-4 (capsules)

| **Ingredients** | **Ex. 1** | **Ex. 2** | **Ex. 3** | **Ex. 4** |
|---|---|---|---|---|
| **Stage-A (Blending & Compaction)** | **mg/cap** | | | |
| Pirfenidone | 267.267 | 267.267 | 267.267 | 267.000 |
| Croscarmellose Sodium (*Ac-Di-Sol*®) | 28.000 | 16.000 | 22.000 | 19.000 |
| Isomalt (Galen IQ 721) | 27.733 | 27.733 | 27.733 | 28.000 |

| **Stage**- **B** (**Lubrication**) | | | | |
|---|---|---|---|---|
| Magnesium Stearate (Ligamed® MF-2-V) | 1.000 | 1.000 | 1.000 | 1.000 |
| ***Fill weight (mg)*** | **324.000** | **312.000** | **318.000** | **315.000** |
| **Stage**-**C** (**Encapsulation**) | | | | |
| Hard Gelatin Capsule Shells *(Size "1")* | 75.000 | 75.000 | 75.000 | 75.000 |
| ***Capsule weight (mg)*** | **399.000** | **387.000** | **393.000** | **390.000** |
| | | | | |

| **Physical Parameters (Blend)** | | | | |
|---|---|---|---|---|
| Dᵥ90 of pirfenidone drug substance | 145 | 145 | 145 | 145 |
| Bulk Density (g/ml) | 0.556 | 0.573 | 0.548 | 0.553 |
| Tapped Density (g/ml) | 0.798 | 0.796 | 0.807 | 0.798 |
| Compressibility Index (%) | 30.357 | 28.000 | 32.099 | 30.702 |
| Hausner's Ratio | 1.436 | 1.389 | 1.473 | 1.443 |

| **Physical Parameters (Capsules)** | | | | |
|---|---|---|---|---|
| Average Lock Length (mm) | 18.98 | 18.95 | 19.01 | 19.00 |
| Average Weight (mg) | 399.8 | 386.5 | 393.7 | 390.6 |
| Disintegration time (min) | 2-3 min | 2-3 min | 2-3 min | 2-3 min |

### Procedure:

The dispensed quantities of stage A materials are sifted through a suitable sieve (e.g. #30 mesh) and blended for a suitable time (e.g. 5 minutes) in a blender. The sifted materials are compacted using roller compactor with suitable set of parameters. The compacts are milled to the required sized granules (e.g. approx. 700 µm). Milled granules were sifted through a suitable sieve (e.g. #25 mesh) and the sized granules are blended with extra granular materials of stage B, in blender for a suitable time (for example 5 minutes). The lubricated granules are filled into size 1 hard gelatin capsules.

| **Example** | **Mean Cumulative % Labelled Amount Dissolved %]** | | | | | | |
|---|---|---|---|---|---|---|---|
| | **5 min** | **10 min** | **15 min** | **20 min** | **30 min** | **45 min** | **60 min** |
| Esbriet® 267 mg capsules (Lot no. 3056759) | 58 | 78 | 84 | 90 | 98 | 102 | 103 |
| Example 1 | 64 | 81 | 91 | 96 | 100 | 101 | 101 |
| Example 2 | 56 | 75 | 87 | 93 | 99 | 101 | 102 |
| Example 3 | 60 | 78 | 88 | 93 | 99 | 101 | 101 |
| Example 4 | 50 | 66 | 76 | 84 | 93 | 98 | 99 |

### Examples 5-7 (tablets)

| **Ingredients** | **Ex. 5** | **Ex. 6** | **Ex. 7** |
|---|---|---|---|
| **Stage-A (Sifting & Blending)** | **mg/tab** | | |
| Pirfenidone | 810.388 | 803.007 | 803.007 |
| Mannitol (Pearlitol® SD200) | 195.612 | 115.993 | 104.993 |
| Sodium Starch Glycolate (Explotab®) | 15.000 | - | - |

| **Stage-B (Binder Solution)** | | | |
|---|---|---|---|
| Hypromellose (Methocel® E15 Premium LV) | 18.000 | 24.000 | 28.000 |
| Water, Purified | q.s. | q.s. | q.s. |

| **Stage-C (Blending & Lubrication)** | | | |
|---|---|---|---|
| Sodium Starch Glycolate (Explotab®) | 9.000 | 20.000 | 16.000 |
| Silica Colloidal Anhydrous (Aerosil® 200) | 6.000 | 6.000 | 6.000 |
| Sodium Stearyl Fumarate | - | 6.000 | 6.000 |
| Magnesium Stearate | 6.000 | - | - |
| ***Core Tablet Weight (mg)*** | ***1060.000*** | ***975.000*** | ***964.000*** |

| **Stage-D (Coating)** | | | |
|---|---|---|---|
| Opadry® Clear 06F590004 | 30.000 | 25.000 | 25.000 |
| Water, Purified | q.s. | q.s. | q.s. |
| ***Coated Tablet Weight (mg)*** | ***1090.000*** | ***1000.000*** | ***989.000*** |

### Procedure:

The stage-A materials were sifted through a suitable sieve (e.g. #30 mesh), loaded into a rapid mixer granulator and mixed for a suitable time. The binder solution was prepared by dispersing binder of stage-B into the purified water. The binder solution was slowly added into the rapid mixer granulator to obtain a wet mass. The wet mass was dried in Fluidized bed drier. The dried granules were milled. The sized granules were sifted through a suitable sieve (e.g. #25 mesh) and blended with sifted extra granular materials of Stage-C. The resulting mixture was compressed to (core) tablets in a tabletting machine and film-coated (using stage-D film coating) in an automated coating machine with perforated pan.

| **Blend Parameters** | **Ex. 5** | **Ex. 6** | **Ex. 7** |
|---|---|---|---|
| Bulk Density (g/ml) | 0.468 | 0.451 | 0.490 |
| Tapped Density (g/ml) | 0.570 | 0.552 | 0.610 |
| Compressibility Index (%) | 17.895 | 18.29 | 21.428 |
| Hausner's Ratio | 1.218 | 1.223 | 1.273 |

| **Tablet Core Parameters** | **Ex. 5** | **Ex. 6** | **Ex. 7** |
|---|---|---|---|
| Tablet Weight (mg) | 1060-1064 | 965-975 | 960-970 |
| Hardness (Newtons) | 150-156 | 145-155 | 140-150 |
| Thickness (mm) | 6.39-6.41 | 6.00-6.10 | 6.60-6.70 |
| Disintegration Time (min) | 2 min 0 sec | 2 min 05 sec | 3 min 40 sec |
| Friability (%) | 0.03 | 0.061 | 0 |

| **Coated Tablet Parameters** | **Ex. 5** | **Ex. 6** | **Ex. 7** |
|---|---|---|---|
| Tablet Weight (mg) | 980-990 | 980-990 | 980-990 |
| Thickness (mm) | 6.80-6.90 | 6.70-6.80 | 6.80-6.90 |
| Disintegration Time (min) | 7 min 38 sec | 8-9 min | 7 min 38 sec |

| **Example** | **Mean Cumulative % Labelled Amount Dissolved [%]** | | | | | | |
|---|---|---|---|---|---|---|---|
| | **5 min** | **10 min** | **15 min** | **20 min** | **30 min** | **45 min** | **60 min** |
| **Example 5** | 59 | 91 | 96 | 99 | 103 | 105 | 105 |
| **Example 6** | 59 | 86 | 92 | 95 | 96 | 97 | 97 |
| **Example 7** | 44 | 75 | 90 | 95 | 99 | 101 | 102 |

### Examples 8 and 9 (tablets)

| **Ingredients** | **Ex. 8** | **Ex. 9** |
|---|---|---|
| **Stage-A (Dispensing & Blending)** | **mg/tab** | |
| Pirfenidone | 802.605 | 803.893 |
| Mannitol (Pearlitol® 200 SD) | 106.395 | 105.107 |
| Sodium Starch Glycolate (Explotab®) | - | 8.000 |

| **Stage-B (Binder Solution)** | | |
|---|---|---|
| Povidone (Plasdone® K-29/32) | 25.000 | 25.000 |
| Water, Purified | q.s. | q.s. |

| **Stage-C (Blending & Lubrication)** | | |
|---|---|---|
| Sodium Starch Glycolate (Explotab®) | 16.000 | 8.000 |
| Silica, Colloidal Anhydrous (Aerosil® 200 Pharma) | 6.000 | 6.000 |
| Sodium Stearyl Fumarate | 6.000 | 6.000 |
| ***Core tablet weight (mg)*** | **962.000** | **962.000** |
| **Stage-D (Coating)** | | |
| Opadry® Pink OY-S-34907 | 25.000 | 25.000 |
| Water, Purified | q.s. | q.s. |
| ***Coated Tablet weight (mg)*** | **987.000** | **987.000** |

### Procedure:

The stage-A materials were sifted through a suitable sieve (e.g. #30 mesh), loaded into a rapid mixer granulator and mixed for a suitable time. The binder solution was prepared by dispersing binder of stage-B into the purified water. The binder solution was slowly added into the rapid mixer granulator to obtain a wet mass. The wet granules were dried. The dried granules were milled. The sized granules were sifted through a suitable sieve (e.g. #25 mesh) and blended with sifted extra granular materials of Stage-C. The resulting mixture was compressed to (core) tablets in a tabletting machine and film-coated (using stage-D film coating) in an automated coating machine with perforated pan.

| **Blend Parameters** | **Ex. 8** | **Ex. 9** |
|---|---|---|
| Bulk Density (g/ml) | 0.526 | 0.490 |
| Tapped Density (g/ml) | 0.623 | 0.610 |
| Compressibility Index (%) | 16 | 21.428 |
| Hausner's Ratio | 1.18 | 1.273 |

| **Tablet Core Parameters** | **Ex. 8** | **Ex. 9** |
|---|---|---|
| Tablet Weight (mg) | 960-970 | 960-970 |
| Hardness (Newtons) | 140-155 | 160-175 |
| Thickness (mm) | - | 6.55-6.65 |
| Disintegration Time (min) | 2 min 30 sec | 3-4 min |
| Friability (%) | 0 | 0 |

| **Coated Tablet Parameters** | **Ex. 8** | **Ex. 9** |
|---|---|---|
| Tablet Weight (mg) | 980-990 | 980-990 |
| Thickness (mm) | - | 6.70-6.80 |
| Disintegration Time (min) | 5-6 min | 8-9 min |

| **Example** | **Mean Cumulative % Labelled Amount Dissolved [%]** | | | | | | |
|---|---|---|---|---|---|---|---|
| | **5 min** | **10 min** | **15 min** | **20 min** | **30 min** | **45 min** | **60 min** |
| Example 8 | 72 | 97 | 102 | 103 | 103 | 103 | 103 |
| Example 9 | 33 | 83 | 98 | 100 | 100 | 102 | 101 |

### Examples 10 and 11 (tablets)

| **Ingredients** | **Ex. 10** | **Ex. 11** |
|---|---|---|
| **Stage-A (Sifting & Blending)** | **mg/tab** | |
| Pirfenidone | 803.893 | 801.802 |
| Mannitol (Pearlitol® SD 200) | 50.000 | 30.000 |
| Microcrystalline Cellulose (Pharmacel® 101) | 51.107 | - |
| Croscarmellose Sodium (Ac-di-sol®) | 10.000 | 5.000 |

| **Stage-B (Binder Solution)** | | |
|---|---|---|
| Povidone (Plasdone® K-29/32) | 35.000 | 20.000 |
| Water, Purified | q.s. | q.s. |

| **Stage-C (Blending & Lubrication)** | | |
|---|---|---|
| Microcrystalline Cellulose (Pharmacel®101) | - | 40.198 |
| Croscarmellose Sodium (Ac-di-sol®) | 10.000 | 15.000 |
| Silica, Colloidal Anhydrous (Aerosil® 200 Pharma) | 3.000 | 3.000 |
| Sodium Stearyl Fumarate | 6.000 | 6.000 |
| ***Core Tablet Weight (mg)*** | **969.000** | **921.000** |

| **Stage-D (Coating)** | | |
|---|---|---|
| Opadry® II Purple 85F500083 | 27.000 | 24.000 |
| Water, Purified | q.s. | q.s. |
| ***Coated Tablet Weight (mg)*** | **996.000** | **945.000** |

### Procedure:

The stage-A materials were sifted through a suitable sieve (e.g. #30 mesh), loaded into a rapid mixer granulator and mixed for a suitable time. The binder solution was prepared by dispersing binder of stage-B into the purified water. The binder solution was slowly added into the rapid mixer granulator to obtain a wet mass. The wet mass was milled and the sized granules were dried. The dried granules were sifted through a suitable sieve (e.g. #20 mesh) and blended with sifted extra granular materials of Stage-C. The resulting mixture was compressed to (core) tablets in a tabletting machine and film-coated (using stage-D film coating) in an automated coating machine with perforated pan.

| **Blend Parameters** | **Ex. 10** | **Ex. 11** |
|---|---|---|
| Bulk Density (g/ml) | 0.440 | 0.449 |
| Tapped Density (g/ml) | 0.551 | 0.592 |
| Compressibility Index (%) | 20.19 | 24.15 |
| Hausner's Ratio | 1.252 | 1.318 |

| **Tablet Core Parameters** | **Ex. 10** | **Ex. 11** |
|---|---|---|
| Tablet Weight (mg) | 966-977 | 917-924 |
| Hardness (Newtons) | 166-175 | 137-156 |
| Thickness (mm) | 6.69-6.72 | 6.95-7.02 |
| Disintegration Time (min) | 3 min | 0 min 36 sec |
| Friability (%) | 0.09 | 0 |

| **Coated Tablet Parameters** | **Ex. 10** | **Ex. 11** |
|---|---|---|
| Tablet Weight (mg) | 995-1000 | 940-950 |
| Thickness (mm) | 6.77-6.79 | 7.00-7.05 |
| Disintegration Time (min) | 4-5 min | 1 min |

| **Example** | **Mean Cumulative % Labelled Amount Dissolved [%]** | | | | | | |
|---|---|---|---|---|---|---|---|
| | **5 min** | **10 min** | **15 min** | **20 min** | **30 min** | **45 min** | **60 min** |
| Example 10 | 53 | 94 | 97 | 98 | 99 | 99 | 99 |
| Example 11 | 93 | 99 | 100 | 101 | 102 | 102 | 103 |

### Examples 12-15 (tablets)

| **Ingredients** | **Ex. 12 267 mg** | **Ex. 13 267 mg** | **Ex. 14 534 mg** | **Ex. 15 801 mg** |
|---|---|---|---|---|
| **Stage-A (Sifting)** | **mg/tab** | | | |
| Pirfenidone | 267.000 | 267.964 | 534.000 | 801.000 |
| Mannitol (Pearlitol® SD 200) | 10.000 | 34.369 | 20.000 | 30.000 |
| Croscarmellose Sodium | 1.667 | - | 3.334 | 5.000 |
| Sodium Starch Glycolate (Primogel®) | - | 2.667 | - | - |

| **Stage-B (Binder Solution)** | | | | |
|---|---|---|---|---|
| Povidone (Plasdone® K-29/32) | 6.667 | 8.333 | 13.334 | 20.000 |
| Water, Purified | q.s. | q.s. | q.s. | q.s. |

| **Stage-C (Blending & Lubrication)** | | | | |
|---|---|---|---|---|
| Microcrystalline Cellulose (Comprecel/ Pharmacel® PH 101) | 13.666 | - | 27.332 | 41.000 |
| Sodium Starch Glycolate (Primogel®) | - | 2.667 | - | - |
| Croscarmellose Sodium | 5.000 | - | 10.000 | 15.000 |
| Silica, Colloidal Anhydrous | 1.000 | 2.000 | 2.000 | 3.000 |
| Sodium Stearyl Fumarate | 2.000 | 2.000 | 4.000 | 6.000 |
| ***Core Tablet Weight (mg)*** | **307.000** | **320.000** | **614.000** | **921.000** |

| **Stage-D (Coating)** | | | | |
|---|---|---|---|---|
| Opadry® Yellow 85F520311 | 8.000 | - | - | - |
| Opadry® II Brown 85F565071 | - | 8.000 | - | - |
| Opadry® Orange 85F530178 | - | - | 16.000 | - |
| Opadry® II Purple 85F500083 | - | - | - | 24.000 |
| Water, Purified | q.s. | q.s. | q.s. | q.s. |
| ***Coated Tablet Weight (mg)*** | **315.000** | **328.000** | **630.000** | **945.000** |

### Procedure:

The stage-A materials were sifted through a suitable sieve (e.g. # 30 mesh), loaded into a rapid mixer granulator and mixed for a suitable time (e.g. 5 minutes). The binder solution was prepared by dispersing binder of stage-B into the purified water under stirring. The binder solution was slowly added into the rapid mixer granulator to obtain a wet mass. The wet mass was milled through a 6350 µm screen and the wet granules were dried. The dried granules were sifted through a suitable sieve (e.g. # 20 mesh) and retains collected were milled through a 1016 µm screen. The sized granules blended with extra granular materials of Stage-C (sifted in advance through a suitable sieve, e.g. # 30 mesh), The resulting mixture was compressed to (core) tablets in a tabletting machine. The coating solution was prepared by dispersing the coating material (stage-D) directly in to the purified water under stirring (20% w/w solids) and stirring was continued for a suitable time (e.g. 45 minute). The (core) tablets were coated with the coating solution by using an automated coating machine with perforated pan.

| **Blend Parameters** | **Ex. 12** | **Ex. 13** | **Ex. 14** | **Ex. 15** |
|---|---|---|---|---|
| Dv90 of pirfenidone drug substance | 145 | 150 | 145 | 145 |
| Bulk Density (g/ml) | 0.449 | 0.497 | 0.449 | 0.424 |
| Tapped Density (g/ml) | 0.592 | 0.644 | 0.592 | 0.538 |
| Compressibility Index (%) | 24.15 | 22.82 | 24.15 | 21.212 |
| Hausner's Ratio | 1.318 | 1.295 | 1.318 | 1.269 |

| **Tablet Core Parameters** | **Ex. 12** | **Ex. 13** | **Ex. 14** | **Ex. 15** |
|---|---|---|---|---|
| Tablet Weight (mg) | 307 | 320 | 614.5 | 923.5 |
| Hardness (Newtons) | 90 | 95-105 | 110 | 166 |
| Thickness (mm) | 5.30 | 5.06 | 6.82 | 6.93 |
| Disintegration Time (min) | 30 sec | 2-3 | 34 sec | 2 |
| Friability (%) | 0.09 | 0 | 0 | 0 |

| **Coated Tablet Parameters** | **Ex. 12** | **Ex. 13** | **Ex. 14** | **Ex. 15** |
|---|---|---|---|---|
| Tablet Weight (mg) | 315 | 325-331 | 630.5 | 947.5 |
| Thickness (mm) | 5.35 | 5.13 | 6.83 | 7.00 |
| Disintegration Time (min) | 1 | 5-6 | 1 | 3 |

| **Example** | **Mean Cumulative % Labelled Amount Dissolved [%]** | | | | | | |
|---|---|---|---|---|---|---|---|
| | **5 min** | **10 min** | **15 min** | **20 min** | **30 min** | **45 min** | **60 min** |
| Example 12 | 90 | 97 | 101 | 102 | 102 | 103 | 103 |
| Example 13 | 56 | 92 | 98 | 100 | 101 | 101 | 101 |
| Example 14 | 93 | 97 | 100 | 101 | 101 | 102 | 102 |
| Example 15 | 81 | 91 | 93 | 95 | 97 | 99 | 100 |

### Examples 16-18 (tablets)

| **Ingredients** | **Ex. 16** | **Ex. 17** | **Ex. 18** |
|---|---|---|---|
| **Stage-A (Sifting)** | **mg/tab** | | |
| Pirfenidone | 267.454^{∗} | 534.909^{∗} | 802.364^{∗} |
| Mannitol (Pearlitol® 200 SD) | 8.333 | 16.667 | 25.000 |
| Croscarmellose Sodium (Ac-Di-Sol® SD-711) | 3.333 | 6.667 | 10.000 |

| **Stage-B (Binder Solution)** | | | |
|---|---|---|---|
| Povidone (Plasdone™ K-29/32) | 6.667 | 13.334 | 20.000 |
| Water, Purified | q.s. | q.s. | q.s. |

| **Stage-C (Blending & Lubrication)** | | | |
|---|---|---|---|
| Microcrystalline Cellulose (Pharmacel® 101) | 13.212^{∗} | 26.424^{∗} | 39.636^{∗} |
| Croscarmellose Sodium (Ac-Di-Sol® SD-711) | 5.000 | 10.000 | 15.000 |
| Colloidal Silicon Dioxide (Aerosil® 200 Pharma) | 1.000 | 2.000 | 3.000 |
| Sodium Stearyl Fumarate | 2.000 | 4.000 | 6.000 |
| ***Core Tablet Weight (mg)*** | **307.000** | **614.000** | **921.000** |
| **Stage-D (Coating)** | | | |
| Opadry® II Yellow 85F520311 | 8.000 | - | - |
| Opadry® II Orange 85F530178 | - | 16.000 | - |
| Opadry® II Purple 85F500083 | - | - | 24,000 |
| Water, Purified | q.s. | q.s. | q.s. |
| ***Coated Tablet Weight (mg)*** | **315.000** | **630.000** | **945.000** |

| | | | |
|---|---|---|---|
| ^{∗}Adjusted for drug assay and water content (Theoretical weight of pirfenidone = 267.000 mg, 534.000 mg and 801.000 mg and of microcrystalline cellulose = 13.667 mg, 27.332 mg and 41.000 mg). | | | |

### Procedure:

The stage-A materials were sifted through a suitable sieve (e.g. # 30 mesh), loaded into a rapid mixer granulator and mixed for a suitable time (e.g. 5 minutes). The binder solution was prepared by dispersing binder of stage-B into the purified water under stirring. The binder solution was slowly added into the rapid mixer granulator to obtain a wet mass. The wet mass was milled through a 6350 µm screen and the wet granules were dried. The dried granules were sifted through a suitable sieve (e.g. # 20 mesh) and retains collected were milled through a 1016 µm screen. The sized granules blended with extra granular materials of Stage-C (sifted in advance through a suitable sieve, e.g. # 30 mesh). The resulting mixture was compressed to (core) tablets in a tabletting machine. The coating solution was prepared by dispersing the coating material (stage-D) directly in to the purified water under stirring (20 % w/w solids) and stirring was continued for a suitable time (e.g. 45 minute). The (core) tablets were coated with the coating solution by using an automated coating machine with perforated pan.

| **Blend Parameters** | **Ex. 16** | **Ex. 17** | **Ex. 18** |
|---|---|---|---|
| Dᵥ90 of pirfenidone drug substance | 145 µm | 145 µm | 145 µm |
| Bulk Density (g/ml) | 0.433 | 0.406 | 0.433 |
| Tapped Density (g/ml) | 0.552 | 0.522 | 0.552 |
| Compressibility Index (%) | 21.67 | 22.22 | 21.67 |
| Hausner's Ratio | 1.276 | 1.286 | 1.276 |

| **Tablet Core Parameters** | **Ex. 16** | **Ex. 17** | **Ex. 18** |
|---|---|---|---|
| Average Tablet Weight (mg) | 307.6 | 614.4 | 921.5 |
| Hardness (Newtons) | 82-92 | 112-115 | 138-152 |
| Thickness (mm) | 5.18-5.25 | 6.69-6.72 | 6.98-7.02 |
| Disintegration Time | 36 sec | 42 sec | 50 sec |
| Friability (%) | 0 | 0.03 | 0 |

| **Coated Tablet Parameters** | **Ex. 16** | **Ex. 17** | **Ex. 18** |
|---|---|---|---|
| Average Tablet Weight (mg) | 314.8 | 628.6 | 947 |
| Thickness (mm) | 5.27-5.29 | 6.80-6.83 | 7.03-7.09 |
| Disintegration Time (min) | 1 min 20 sec | 1 min | 1.5 min |

| **Example** | **Mean Cumulative % Labelled Amount Dissolved [%]** | | | | | |
|---|---|---|---|---|---|---|
| | **5 min** | **10 min** | **15 min** | **20 min** | **30 min** | **45 min** |
| Esbriet® tablets 267 mg (Lot no. M0009M7) | 73 | 94 | 98 | 100 | 101 | 100 |
| Example 16 | 85 | 91 | 96 | 98 | 99 | 99 |
| Example 17 | 92 | 95 | 97 | 99 | 99 | 100 |
| Esbriet® tablets 801 mg (Lot no. M1000M1) | 33 | 86 | 93 | 96 | 98 | 101 |
| Example 18 | 80 | 99 | 101 | 102 | 102 | 102 |

### Comparative Example 1 (capsule)

| **Ingredients** | **Comp. Ex. 1** |
|---|---|
| **Stage** - **A (Dry mix)** | **mg/tab** |
| Pirfenidone | 267.481 |
| Microcrystalline Cellulose (Avicel® PH 102) | 23.519 |
| Croscarmellose Sodium (Ac-Di-Sol® SD 711) | 26.500 |

| **Stage** - **B (Granulation)** | |
|---|---|
| Povidone (Plasdone® K 29/32) | 6.000 |
| Water, Purified | q. s. |

| **Stage** - **C (Blending & Lubrication)** | |
|---|---|
| Magnesium Stearate | 1.500 |
| ***Fill weight (mg)*** | **325.000** |

| **Stage** - **D (Encapsulation)** | |
|---|---|
| Hard Gelatin Capsule Shells (Size "1") | 96.000 |
| ***Capsule weight (mg)*** | **421.000** |
| | |

| **Physical Parameters (granules)** | |
|---|---|
| Bulk Density (g/ml) | 0.422 |
| Tapped Density (g/ml) | 0.552 |
| Compressibility Index (%) | 23.040 |
| Hausner's Ratio | 1.305 |

### Procedure:

The stage-A materials were sifted through a suitable sieve (e.g. #30 mesh), loaded into a rapid mixer granulator and mixed for a suitable time. The binder solution was prepared by dispersing binder of stage-B into the purified water. The binder solution was slowly added into the rapid mixer granulator to obtain a wet mass. The wet granules were dried. The dried granules were milled. The sized granules were sifted through a suitable sieve (e.g. #30 mesh) and blended with sifted extra granular materials of Stage-C for a suitable time. The lubricated blend could not be filled into size "1" capsules. Thus, the dry-granulation process is preferred.

### Comparative Examples 2-4 (tablets)

| **Ingredients** | **Comp. Ex. 2** | **Comp. Ex. 3** | **Comp. Ex. 4** |
|---|---|---|---|
| **Stage** - **A (Blending)** | **mg/tab** | | |
| Pirfenidone | 268.018 | 268.018 | 268.018 |
| Mannitol (Pearlitol® SD 200) | 42.982 | 64.982 | - |
| Dicalcium Phosphate Dihydrate | - | - | 67.982 |
| Hypromellose (Methocel® E5 Premium LV) | 6.000 | - | 6.000 |
| Xanthan Gum | - | 7.000 | - |
| Sodium Starch Glycolate (Explotab®) | 6.000 | 8.000 | 6.000 |
| Sodium Stearyl Fumarate | 2.000 | 2.000 | - |
| Magnesium Stearate | - | - | 2.000 |
| ***Core tablet weight (mg)*** | **325.000** | **350.000** | **350.000** |

### Procedure:

The dispensed quantities were sifted through suitable sieve (e.g. #30 mesh). The sifted materials were blended for a suitable time in a blender. The resulting mixture was compressed to core tablets on a tabletting machine.

The desired hardness (above 60 N) could not be achieved. The direct compression process failed to give the desired hardness for the tablets.

### Comparative Examples 5 and 6 (tablets)

| **Ingredients** | **Comp. Ex. 5** | **Comp. Ex. 6** |
|---|---|---|
| **Stage** - **A (Sifting & Blending)** | **mg/tab** | |
| Pirfenidone | 268.018 | 268.018 |
| Dicalcium Phosphate Dihydrate (DICOFOS® D014) | 67.982 | 67.982 |
| Sodium Starch Glycolate | 5.000 | 5.000 |

| **Stage-B (Binder Solution)** | | |
|---|---|---|
| Hypromellose (Methocel® E5 Premium LV) | 6.000 | 6.000 |
| Water, Purified | q.s. | q.s. |

| **Stage-C (Blending & Lubrication)** | | |
|---|---|---|
| Sodium Starch Glycolate | 3.000 | 3.000 |
| Silica, Colloidal Anhydrous (Aerosil® 200 Pharma) | - | 1.800 |
| Magnesium Stearate | 2.000 | 2.000 |
| ***Core tablet weight (mg)*** | **352.000** | **353.800** |

### Procedure:

The stage-A materials were sifted through a suitable sieve (e.g. #30 mesh), loaded into a rapid mixer granulator and mixed for a suitable time. The binder solution was prepared by dispersing binder of stage-B into the purified water. The binder solution was slowly added into the rapid mixer granulator to obtain a wet mass. The wet mass was dried in fluidized bed drier. The dried granules were sifted through a suitable sieve (e.g. #25 mesh) and retains were milled. The sized granules were blended with sifted extra granular materials of Stage-C. The resulting mixture was compressed to core tablets in a tabletting machine.

The desired hardness (above 60 N) could not be achieved without a sugar alcohol present in the granules. Maximum hardness achieved for both the batches was 45 N.

### Comparative Examples 7-8 (tablets)

| **Ingredients** | **Comp. Ex. 7** | **Comp. Ex. 8** |
|---|---|---|
| **Stage-A (Dispensing & Blending)** | **mg/tab** | |
| Pirfenidone | 803.007 | 803.893 |
| Microcrystalline Cellulose (Comprecel® M 102 D) | 104.993 | 103.107 |
| Croscarmellose Sodium (Ac-Di-sol®) | - | 8.000 |

| **Stage-B (Binder solution)** | | |
|---|---|---|
| Povidone (Plasdone® K-29/32) | 28.000 | 35.000 |
| Water, Purified | q.s. | q.s. |

| **Stage-C (Blending & Lubrication)** | | |
|---|---|---|
| Croscarmellose Sodium (Ac-Di-sol®) | 16.000 | 8.000 |
| Silica, Colloidal Anhydrous (Aerosil® 200 Pharma) | 6.000 | 6.000 |
| Sodium Stearyl Fumarate | 6.000 | 6.000 |
| ***Core tablet weight (mg)*** | **964.000** | **970.000** |

### Procedure:

The stage-A materials were sifted through a suitable sieve (e.g. #30 mesh), loaded into a rapid mixer granulator and mixed for a suitable time. The binder solution was prepared by dispersing binder of stage-B into the purified water. The binder solution was slowly added into the rapid mixer granulator to obtain a wet mass. The wet mass was dried in fluidized bed drier. The dried granules were sifted through a suitable sieve (e.g. #25 mesh) and retains were milled. The sized granules were blended with sifted extra granular materials of Stage-C. The resulting mixture was compressed to core tablets in a tabletting machine.

| **Blend Parameters** | **Comp. Ex. 7** | **Comp. Ex. 8** |
|---|---|---|
| Bulk Density (g/ml) | 0.411 | 0.414 |
| Tapped Density (g/ml) | 0.532 | 0.506 |
| Compressibility Index (%) | 22.727 | 18.293 |
| Hausner's Ratio | 1.294 | 1.224 |

| **Tablet Core Parameters** | **Comp. Ex. 7** | **Comp. Ex. 8** |
|---|---|---|
| Tablet Weight (mg) | 960-970 | 970-980 |
| Hardness (Newtons) | 90-100 | 90-100 |
| Thickness (mm) | 6.90-7.00 | 6.80-6.90 |
| Disintegration Time (min) | 0 min 24 sec | 0 min 50 sec |
| Friability (%) | 0.4 | 3.2 |

The desired hardness (above 120 N) could not be achieved without a sugar alcohol present in the granules.

## Claims

1. Granules containing pirfenidone, a sugar alcohol, optionally a disintegrant and optionally a further pharmaceutical excipient, wherein the weight ratio of the sugar alcohol to pirfenidone is 2 : 100 to 30 : 100.

2. The granules according to claim 1, wherein the granules do not contain a glidant.

3. The granules according to claim 1 or 2, wherein the pharmaceutical excipient is selected from a binder and a filler.

4. The granules according to claim 3, wherein the granules consist of pirfenidone, a disintegrant, a sugar alcohol, and optionally a binder and optionally a filler.

5. The granules according to any one of the preceding claims, wherein the sugar alcohol is a C₄₋₁₂ sugar alcohol.

6. The granules according to claim 5, wherein the sugar alcohol is selected from erythritol, xylitol, sorbitol, mannitol, maltitol, lactitol and isomalt.

7. The granules according to claim 6, wherein the sugar alcohol is mannitol or isomalt.

8. A tablet or capsule for oral administration containing the granules according to any one of the preceding claims.

9. Process for preparing a capsule containing pirfenidone, a sugar alcohol, optionally a disintegrant and optionally a further pharmaceutical excipient, comprising the steps:
i) preparing a mixture containing pirfenidone, a sugar alcohol, optionally a first disintegrant and optionally a further first pharmaceutical excipient,
ii) subjecting the mixture obtained in step (i) to compaction,
iii) milling the compacted material obtained in step (ii) to obtain granules, wherein the weight ratio of the sugar alcohol to pirfenidone in the obtained granules is 2 : 100 to 30 : 100,
iv) optionally mixing the granules with a second disintegrant and/or a further second pharmaceutical excipient, and
v) filling the granules obtained in step (iii) or the mixture obtained in step (iv) into a capsule.

10. The process according to claim 9, wherein the compaction is a roller compaction or performed by slugging.

11. The process according to claim 9 or 10, wherein the capsule contains a mixture consisting of granules made from pirfenidone, a disintegrant and a sugar alcohol, and a lubricant, comprising the steps:
i) preparing a mixture consisting of pirfenidone, a disintegrant and a sugar alcohol,
ii) subjecting the mixture obtained in step (i) to compaction,
iii) milling the compacted material obtained in step (ii) to obtain granules,
iv) mixing the granules obtained in step (iii) with a lubricant, and
v) filling the mixture obtained in step (iv) into a capsule.

12. The process according to claim 11, wherein the disintegrant is croscarmellose sodium, the sugar alcohol is isomalt and the lubricant is magnesium stearate.

13. Process for preparing an optionally film-coated tablet containing pirfenidone, a sugar alcohol, optionally a disintegrant and optionally a further pharmaceutical excipient, comprising the steps:
i) preparing a mixture containing pirfenidone, a sugar alcohol, optionally a first disintegrant and optionally a further first pharmaceutical excipient,
ii) subjecting the mixture obtained in step (i) to wet granulation using an aqueous granulating liquid, wherein the weight ratio of the sugar alcohol to pirfenidone in the obtained granules is 2 : 100 to 30 : 100,
iii) optionally mixing the granules obtained in step (ii) with a second disintegrant and/or a further second pharmaceutical excipient,
iv) subjecting the granules obtained in step (ii) or the mixture obtained in step (iii) to compression to obtain the tablet.

14. The process according to claim 13, wherein the first pharmaceutical excipient is a filler, the aqueous granulating liquid contains a binder and the second pharmaceutical excipient is selected from a filler, a glidant and a lubricant.

15. The process according to claim 14, wherein the tablet or the tablet core consists of a mixture consisting of granules made from pirfenidone, a first disintegrant, a sugar alcohol, a binder and optionally a first filler, a second disintegrant, a second filler, a glidant and a lubricant, comprising the steps:
i) preparing a mixture consisting of pirfenidone, a first disintegrant, a sugar alcohol and optionally a first filler,
ii) subjecting the mixture obtained in step (i) to wet granulation using an aqueous binder-containing solution as granulating liquid,
iii) mixing the granules obtained in step (ii) with a second disintegrant, a second filler, a glidant and a lubricant,
iv) subjecting the mixture obtained in step (iii) to compression to obtain the tablet.

16. The process according to claim 15, wherein the first and second disintegrant is croscarmellose sodium, the sugar alcohol is mannitol, the optionally contained first filler and the second filler are microcrystalline cellulose, the glidant is silicon dioxide and the lubricant is sodium stearyl fumarate.

## Patentansprüche

1. Granulat enthaltend Pirfenidon, einen Zuckeralkohol, gegebenenfalls ein Sprengmittel und gegebenenfalls einen weiteren pharmazeutischen Hilfsstoff, wobei das Gewichtsverhältnis von Zuckeralkohol zu Pirfenidon 2 : 100 bis 30 : 100 beträgt.

2. Das Granulat nach Anspruch 1, wobei das Granulat kein Gleitmittel enthält.

3. Das Granulat nach Anspruch 1 oder 2, wobei der pharmazeutische Hilfsstoff ausgewählt ist aus einem Bindemittel und einem Füllstoff.

4. Das Granulat nach Anspruch 3, wobei das Granulat aus Pirfenidon, einem Sprengmittel, einem Zuckeralkohol und gegebenenfalls einem Bindemittel und gegebenenfalls einem Füllstoff besteht.

5. Das Granulat nach einem der vorhergehenden Ansprüche, wobei der Zuckeralkohol ein C₄₋₁₂ Zuckeralkohol ist.

6. Das Granulat nach Anspruch 5, wobei der Zuckeralkohol ausgewählt ist aus Erythrit, Xylit, Sorbit, Mannit, Maltit, Lactit und Isomalt.

7. Granulat nach Anspruch 6, **dadurch gekennzeichnet, dass** der Zuckeralkohol Mannit oder Isomalt ist.

8. Eine Tablette oder Kapsel zur oralen Verabreichung enthaltend das Granulat nach einem der vorhergehenden Ansprüche.

9. Verfahren zur Herstellung einer Kapsel enthaltend Pirfenidon, einen Zuckeralkohol, gegebenenfalls ein Sprengmittel und gegebenenfalls einen weiteren pharmazeutischen Hilfsstoff, umfassend die Schritte:
i) Herstellen einer Mischung enthaltend Pirfenidon, einen Zuckeralkohol, gegebenenfalls ein erstes Sprengmittel und gegebenenfalls einen weiteren ersten pharmazeutischen Hilfsstoff,
ii) Kompaktieren der in Schritt (i) erhaltenen Mischung,
iii) Mahlen des in Schritt (ii) erhaltenen kompaktierten Materials, um ein Granulat zu erhalten, wobei das Gewichtsverhältnis des Zuckeralkohols zu Pirfenidon im erhaltenen Granulat 2 : 100 bis 30 : 100 beträgt.
iv) gegebenenfalls Mischen des Granulats mit einem zweiten Sprengmittel und/oder einem weiteren zweiten pharmazeutischen Hilfsstoff, und
v) Einfüllen des in Schritt (iii) erhaltenen Granulats oder der in Schritt (iv) erhaltenen Mischung in eine Kapsel.

10. Verfahren nach Anspruch 9, **dadurch gekennzeichnet, dass** die Kompaktierung eine Walzenkompaktierung ist oder durch Verpressen (slugging) erfolgt.

11. Verfahren nach Anspruch 9 oder 10, wobei die Kapsel eine Mischung ist, bestehend aus einem Granulat, hergestellt aus Pirfenidon, einem Sprengmittel und einem Zuckeralkohol, und einem Schmiermittel, umfassend die Schritte:
i) Herstellen einer Mischung bestehend aus Pirfenidon, einem Sprengmittel und einem Zuckeralkohol,
ii) Kompaktieren der in Schritt (i) erhaltenen Mischung,
iii) Mahlen des in Schritt (ii) erhaltenen kompaktierten Materials, um ein Granulat zu erhalten,
iv) Mischen des in Schritt (iii) erhaltenen Granulats mit einem Schmiermittel, und
v) Füllen der in Schritt (iv) erhaltenen Mischung in eine Kapsel.

12. Verfahren nach Anspruch 11, wobei das Sprengmittel Croscarmellose-Natrium, der Zuckeralkohol Isomalt und das Schmiermittel Magnesiumstearat ist.

13. Verfahren zur Herstellung einer gegebenenfalls filmbeschichteten Tablette enthaltend Pirfenidon, einen Zuckeralkohol, gegebenenfalls ein Sprengmittel und gegebenenfalls einen weiteren pharmazeutischen Hilfsstoff, umfassend die Schritte:
i) Herstellen einer Mischung enthaltend Pirfenidon, einen Zuckeralkohol, gegebenenfalls ein erstes Sprengmittel und gegebenenfalls einen weiteren ersten pharmazeutischen Hilfsstoff,
ii) Nassgranulation der in Schritt (i) erhaltenen Mischung unter Verwendung einer wässrigen Granulierflüssigkeit, wobei das Gewichtsverhältnis des Zuckeralkohols zu Pirfenidon im erhaltenen Granulat 2 : 100 bis 30 : 100 beträgt,
iii) gegebenenfalls Mischen des in Schritt (ii) erhaltenen Granulats mit einem zweiten Sprengmittel und/oder einem weiteren zweiten pharmazeutischen Hilfsstoff,
iv) Verpressen des in Schritt (ii) erhaltenen Granulats oder der in Schritt (iii) erhaltenen Mischung, um die Tablette zu erhalten.

14. Verfahren nach Anspruch 13, wobei der erste pharmazeutische Hilfsstoff ein Füllstoff ist, die wässrige Granulierflüssigkeit ein Bindemittel enthält und der zweite pharmazeutische Hilfsstoff ausgewählt ist aus einem Füllstoff, einem Gleitmittel und einem Schmiermittel.

15. Verfahren nach Anspruch 14, wobei die Tablette oder der Tablettenkern aus einer Mischung besteht, die aus einem Granulat, hergestellt aus Pirfenidon, einem ersten Sprengmittel, einem Zuckeralkohol, einem Bindemittel und gegebenenfalls einem ersten Füllstoff, sowie einem zweiten Sprengmittel, einem zweiten Füllstoff, einem Gleitmittel und einem Schmiermittel besteht, umfassend die Schritte:
i) Herstellen einer Mischung bestehend aus Pirfenidon, einem ersten Sprengmittel, einem Zuckeralkohol und gegebenenfalls einem ersten Füllstoff,
ii) Nassgranulation der in Schritt (i) erhaltenen Mischung unter Verwendung einer wässrigen bindemittelhaltigen Lösung als Granulierflüssigkeit,
iii) Mischen des in Schritt (ii) erhaltenen Granulats mit einem zweiten Sprengmittel, einem zweiten Füllstoff, einem Gleitmittel und einem Schmiermittel,
iv) Verpressen der in Schritt (iii) erhaltenen Mischung um die Tablette zu erhalten.

16. Verfahren nach Anspruch 15, wobei das erste und zweite Sprengmittel Croscarmellose-Natrium ist, der Zuckeralkohol Mannit ist, der gegebenenfalls enthaltene erste Füllstoff und der zweite Füllstoff mikrokristalline Cellulose ist, das Gleitmittel Siliziumdioxid ist und das Schmiermittel Natriumstearylfumarat ist.

## Revendications

1. Granules contenant de la pirfénidone, un alcool de sucre, éventuellement un agent délitant et éventuellement un autre excipient pharmaceutique, dans lesquels le rapport pondéral de l'alcool de sucre à la pirfénidone est de 2 : 100 à 30 : 100.

2. Les granulés selon la revendication 1, dans lesquels les granulés ne contiennent pas d'agent glissant.

3. Les granulés selon la revendication 1 ou 2, dans lesquels l'excipient pharmaceutique est choisi parmi un liant et un moyen de remplissage.

4. Les granulés selon la revendication 3, dans lesquels les granulés sont constitués de la pirfénidone, d'un agent délitant, d'un alcool de sucre, et éventuellement d'un liant et éventuellement d'un moyen de remplissage.

5. Les granulés selon l'une quelconque des revendications précédentes, dans lesquels l'alcool de sucre est un alcool de sucre en C₄₋₁₂.

6. Les granulés selon la revendication 5, dans lesquels l'alcool de sucre est choisi parmi l'érythritol, le xylitol, le sorbitol, le mannitol, le maltitol, le lactitol et l'isomalt.

7. Les granulés selon la revendication 6, dans lesquels l'alcool de sucre est le mannitol ou l'isomalt.

8. Comprimé ou capsule pour l'administration orale contenant les granules selon l'une quelconque des revendications précédentes.

9. Procédé de préparation d'une gélule contenant de la pirfénidone, un alcool de sucre, éventuellement un agent délitant et éventuellement un autre excipient pharmaceutique, comprenant les étapes :
i) préparer un mélange contenant de la pirfénidone, un alcool de sucre, éventuellement un premier agent délitant et éventuellement un autre premier excipient pharmaceutique,
ii) soumettre le mélange obtenu à l'étape (i) à un compactage,
iii) broyer le matériau compacté obtenu à l'étape (ii) pour obtenir des granulés, dans lequel le rapport pondéral de l'alcool de sucre à la pirfénidone dans les granulés obtenus est de 2 : 100 à 30 : 100,
iv) éventuellement mélanger les granulés avec un second agent délitant et/ou un autre second excipient pharmaceutique, et
v) remplissage des granulés obtenus à l'étape (iii) ou du mélange obtenu à l'étape (iv) dans une capsule.

10. Procédé selon la revendication 9, dans lequel le compactage est un compactage au presse à comprimés rotative ou réalisé par presser (slugging).

11. Procédé selon la revendication 9 ou 10, dans lequel la capsule contient un mélange constitué de granulés à base de pirfénidone, d'un agent délitant et d'un alcool de sucre, et d'un lubrifiant, comprenant les étapes :
i) préparer un mélange constitué de pirfénidone, d'un agent délitant et d'un alcool de sucre,
ii) soumettre le mélange obtenu à l'étape (i) à un compactage,
iii) broyer le matériau compacté obtenu à l'étape (ii) pour obtenir des granulés,
iv) mélanger les granulés obtenus à l'étape (iii) avec un lubrifiant, et
v) remplissage du mélange obtenu à l'étape (iv) dans une capsule.

12. Procédé selon la revendication 11, dans lequel l'agent délitant est la croscarmellose sodique, l'alcool de sucre est l'isomalt et le lubrifiant est le stéarate de magnésium.

13. Procédé de préparation d'un comprimé éventuellement pelliculé contenant de la pirfénidone, un alcool de sucre, éventuellement un agent délitant et éventuellement un autre excipient pharmaceutique, comprenant les étapes :
i) préparer un mélange contenant de la pirfénidone, un alcool de sucre, éventuellement un premier agent délitant et éventuellement un autre premier excipient pharmaceutique,
ii) soumettre le mélange obtenu à l'étape (i) à une granulation humide en utilisant un liquide de granulation aqueux, dans lequel le rapport pondéral de l'alcool de sucre à la pirfénidone dans les granulés obtenus est de 2 : 100 à 30 : 100,
iii) éventuellement mélanger les granulés obtenus à l'étape (ii) avec un deuxième agent délitant et/ou un autre deuxième excipient pharmaceutique,
iv) soumettre les granules obtenus à l'étape (ii) ou le mélange obtenu à l'étape (iii) à une compression pour obtenir le comprimé.

14. Procédé selon la revendication 13, dans lequel le premier excipient pharmaceutique est un moyen de remplissage, le liquide de granulation aqueux contient un liant et le deuxième excipient pharmaceutique est choisi parmi un moyen de remplissage, un glissant et un lubrifiant.

15. Procédé selon la revendication 14, dans lequel le comprimé ou le noyau de comprimé est constitué d'un mélange constitué de granulés à base de pirfénidone, d'un premier agent délitant, d'un alcool de sucre, d'un liant et éventuellement d'une première moyen de remplissage, d'un deuxième agent délitant, d'une deuxième moyen de remplissage , un glissant et un lubrifiant, comprenant les étapes :
i) préparer un mélange constitué de pirfénidone, d'un premier agent délitant, d'un alcool de sucre et éventuellement d'un premier moyen de remplissage,
ii) soumettre le mélange obtenu à l'étape (i) à une granulation humide en utilisant une solution aqueuse contenant un liant comme liquide de granulation,
iii) mélanger les granulés obtenus à l'étape (ii) avec un deuxième agent délitant, un deuxième moyen de remplissage, un glissant et un lubrifiant,
iv) soumettre le mélange obtenu à l'étape (iii) à une compression pour obtenir le comprimé.

16. Procédé selon la revendication 15, dans lequel le premier et le deuxième agent délitant sont la croscarmellose sodique, l'alcool de sucre est le mannitol, le premier moyen de remplissage et le deuxième moyen de remplissage éventuellement contenues sont la cellulose microcristalline, le glissant est le dioxyde de silicium et le lubrifiant est le stéarylfumarate de sodium.
